# EUROPEAN PATENT SPECIFICATION

(11) **EP 1 537 884 B1**
(45) Date of publication and mention of the grant of the patent: **23.04.2014**
(21) Application number: 03795321.3
(22) Date of filing: 09.09.2003
(51) Int. Cl.: A61L 31/00, A61B 17/00

(54) **EMBOLIZATION DEVICE FOR VESSEL CAVITY IN VIVO**
EMBOLISATIONSVORRICHTUNG FÜR EINE GEFÄSSHÖHLE IN VIVO
DISPOSITIF D'EMBOLISATION POUR CAVITE VASCULAIRE IN VIVO

(30) Priority: 13.09.2002 JP 2002267646
(43) Date of publication of application: 08.06.2005
(73) Proprietor: Kaneka Corporation, Osaka (JP)
(72) Inventor: IWATA, Hiroo, Mishima-gun, Osaka 618-0024 (JP); NISHIDE, Takuji, Otsu-shi, Shiga 520-0105 (JP)
(74) Representative: Gille Hrabal
(86) International application number: PCT/JP2003/011470
(87) International publication number: WO 2004/024207

(56) References cited:
- WO-A1-00/74577
- WO-A1-01/30411
- JP-A- 11 076 249
- JP-A- 2001 299 769
- MURAYAMA Y.: 'A new surface modification technique of platinum coils by ion implantation and protein coating: Use in intravascular treatment of brain aneurysms' NUCLEAR INSTRUMENTS AND METHODS IN PHYSIC RESEARCH 1997, B127/128, pages 1015 - 1018, XP004096894

## Description

### Technical Field

The present invention relates to an embolization device which is placed at a predetermined position in a vessel cavity *in vivo* to embolize the vessel cavity. More particularly, the invention relates to an embolization device which embolizes a blood vessel or a aneurysm formed in a blood vessel.

### Background Art

It is known that cerebrovascular diseases are broadly classified into hemorrhagic lesions, such as subarachnoid hemorrhage and intracerebral hemorrhage, and obstructive lesions caused by atheromatous clots or the like, and that cerebrovascular diseases rapidly develop and have serious prognoses. Above all, subarachnoid hemorrhage is a serious disease with a mortality rate of about 30% within 48 hours of onset. Furthermore, the frequency of rebleeding within two weeks after subarachnoid hemorrhage is 20% to 30%, and in the case of rebleeding, the mortality rate is extremely high at 70% to 90%.

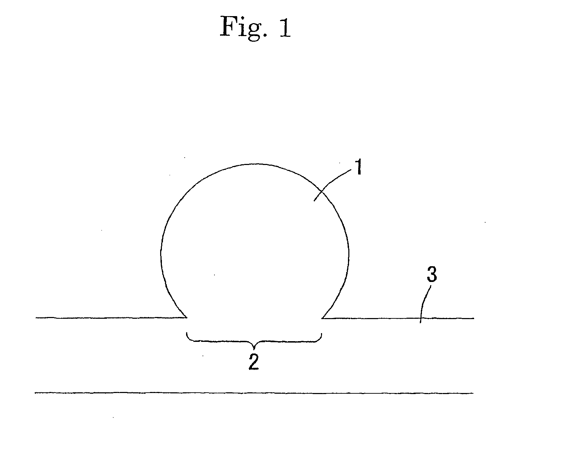

Rupture of cerebral aneurysms, such as a cerebral aneurysm 1 (refer to Fig. 1), is the cause of 80% of all subarachnoid hemorrhages. Ruptured aneurysms are treated surgically to prevent rebleeding, and clipping is the most radical treatment. In the clipping treatment, a craniotomy is performed and then a neck (base) 2 of the cerebral aneurysm (refer to Fig. 1) is clipped to prevent rerupture. However, in the case of high severity, such as deep coma or unstable blood pressure, it is difficult to perform such clipping treatment. Consequently, about only half of patients with subarachnoid hemorrhage caused by rupture of cerebral aneurysms are treated by clipping. Furthermore, clipping is an invasive treatment requiring a craniotomy, and infection associated with the craniotomy is a problem. Moreover, since direct surgery is performed in the clipping treatment, depending on the site of the cerebral aneurysm, there may be a case in which it is difficult to perform a surgical procedure, which is also a problem.

Recently, as a less invasive treatment, vascular embolization, in which, as described in Japanese Patent No. 2880070, an embolization device is percutaneously placed in a cerebral aneurysm to prevent rerupture, has been receiving attention. In the vascular embolization, the embolization device placed in the cerebral aneurysm serves as a physical obstacle to blood flow and thrombi are formed around the embolization device, and thus it is possible to prevent the rerupture of the cerebral aneurysm. As the embolization device to be placed in the cerebral aneurysm, an embolization device comprising a metal coil (hereinafter referred to as an "embolization coil") has been commonly used. Consequently, vascular embolization using an embolization coil is often called "coil embolization". Such an embolization coil is percutaneously guided through a suitable catheter to a cerebral aneurysm and then placed in the cerebral aneurysm by a push-out device detachably connected to the end of the embolization coil. Therefore, the coil embolization can be applied in cases of high severity to which clipping treatment is not applicable and to elderly people.

The coil embolization is performed using X-ray fluoroscopy because it is a percutaneous treatment as described above. In order to achieve visibility by X-ray fluoroscopy, the embolization coil is generally composed of platinum or a platinum alloy.

However, coil embolization is not applicable to the treatment of all ruptured cerebral aneurysms because of its specific problems. For example, when coil embolization is used in a cerebral aneurysm with a large diameter, it is difficult to completely embolize the aneurysm, and compaction of the indwelling embolization coil (coil compaction) easily occurs after treatment, resulting in a high possibility of rebleeding. Furthermore, in the case of a cerebral aneurysm with a wide neck 2 (refer to Fig. 1), the indwelling embolization coil is easily dislodged from the aneurysm to a parent blood vessel 3 (refer to Fig. 1), and it has been pointed out that there is a possibility of complications, such as cerebral infarction, being caused because the thrombi formed on the surface of the dislodged embolization coil flow to peripheries through the bloodstream. Moreover, in the case of a cerebral aneurysm which is formed in a branch of a blood vessel, there is a risk of occlusion in the branch. As described above, although coil embolization is a less invasive treatment, the shape of a cerebral aneurysm for which the coil embolization can be used is limited, and the coil embolization is not yet a technique that is superior to clipping treatment.

Many studies have been conducted using autopsy and animal experiments regarding the tissue response in cerebral aneurysms treated with coil embolization. As a result, it has been found that if an embolization coil is placed in an aneurysm, fibrous tissue is formed by successive cell responses and that the successive cell responses follow the same pattern as that of the wound healing response as shown in Am J Neuroradiol, 1999, 20, 546-548; Neurosurgery, 1998, 43, 1203-1208; Stroke, 1999, 30, 1657-1664; J Neuroradiol, 1999, 26, 7-20; etc.

The wound healing response is believed to include the following five successive steps. Namely, when a wound is caused, blood coagulation and thrombus formation occur due to the activation, adhesion, and aggregation of platelets. Furthermore, activation of the coagulation system and activation of the complement system are initiated. These responses are observed mainly one to two days after the wound has occurred, and are generically referred to as a response in the coagulation/hemostasis phase.

Subsequently, increased blood vessel permeability and vascular dilatation are caused by the actions of histamine, serotonin, prostacyclin, etc. Furthermore, because of PDGF and TGF-β, infiltration and migration of inflammatory cells, such as neutrophils and macrophages, are observed, and lymphocytes appear simultaneously. Phagocytosis of macrophages is initiated, and various cytokines (e.g., PDGF, VEGF, TNF-α, and CSF-1) are secreted from macrophages. These responses are observed mainly one to seven days after the wound has occurred, and are generically referred to as a response in the inflammation phase.

Subsequently, proliferation of fibroblasts is initiated by the actions of cytokines, such as TGF-β and IL-4, derived from macrophages, and synthesis of extracellular matrix and neovascularization are also initiated. These responses are observed mainly three days to two weeks after the wound has occurred, and are generically referred to as a response in the proliferation phase.

Subsequently, tissue reconstruction is carried out by collagen crosslinking, formation of granulation tissue, contraction of the wound, epithelialization, etc. These responses are observed mainly five days to three weeks after the wound has occurred, and are generically referred to as a response in the tissue reconstruction phase.

Lastly, cicatrization and involution of the vascular system occur, and thus the wound healing is completed. These responses are observed mainly two weeks to two years after the wound has occurred, and are generically referred to as a response in the maturation phase.

Platinum, which is a material mainly constituting the embolization coils currently in use, is extremely inactive *in vivo,* and therefore, fibrous tissue formation (organization) does not easily take place in cerebral aneurysms treated with coil embolization. This fact has been pointed out as a limiting factor in the application of coil embolization.

Under such circumstances, prior art techniques have been disclosed to promote thrombus formation around embolization coils.

Namely, in order to enhance thrombus formation on embolization coils having various shapes and properties, such as flexibility, techniques for attaching fibrous members to the embolization coils are disclosed, for example, in Japanese Examined Patent Application Publication No. 7-63508 and Japanese Patent Nos. 2553309, 2682743, 2986409, 3023076, 3024071, and 3085655. However, attachment of a fibrous member to an embolization coil gives rise to problems. For example, the fabrication process becomes complex. Furthermore, since it is difficult to view the fibrous member by X-ray fluoroscopy, there is a possibility that the fibrous member may be dislodged into a parent blood vessel, resulting in complications, such as cerebral infarction. Moreover, the coefficient of friction of the surface of the embolization coil is extremely increased by the attachment of the fibrous member, and operationality during guiding of the embolization coil through a catheter is decreased.

In other prior art techniques, for example, in Japanese Patent Nos. 2620530 and 3016418, inclusion of biocompatible polymers into embolization coils having a specific shape and properties, such as flexibility, is disclosed. However, the biocompatible polymers disclosed are fibrous materials with thrombus-forming properties, and it is evident that there is a possibility of the same problems occurring as those described above. Furthermore, in other prior art techniques, for example, Japanese Patent No. 2908363 and Japanese Unexamined Patent Application Publication No. 11-76249, helical coils each provided with a strand composed of a biologically active material and axially extending therein are disclosed. However, since the strand that can be placed in the embolization coil has an extremely small diameter, it is difficult to produce such a strand. Furthermore, the flexibility of the entire embolization coil is inevitably decreased because of the placement of the strand, and thus there is an unavoidable possibility of causing serious complications, such as perforation of the aneurysm during the placement of the embolization coil.

In view of the problems described above, it is an object of the present invention to readily provide an embolization device which promotes not only thrombus formation but also organization around the embolization device after it has been placed, thus showing a superior embolizing effect compared with the conventional embolization devices.

### Disclosure of Invention

The present inventors have conducted intensive research in order to overcome the problems described above. As a result, an embolization device for embolizing a vessel cavity *in vivo* has been invented, the embolization device being characterized in that it includes a β(1→3) glucan as a biological response modifier (BRM). Herein, the vessel cavity *in vivo* can be a blood vessel or a aneurysm formed in a blood vessel.

The BRM is preferably applied by coating to a surface of the embolization device.

The BRM is a β(1→3) glucan. The β(1→3) glucan is preferably curdlan.

The β(1→3) glucan may have a branch comprising a β(1→6) glucan. The β(1→3) glucan having the branch comprising a β(1→6) glucan is preferably lentinan or sizofiran.

In addition, the embolization device is preferably a coil. More preferably, the coil comprises a metal wire composed of any one of platinum, gold, silver, and tantalum, or an alloy wire containing any one of platinum, gold, silver, and tantalum in an amount of 80% by weight or more.

### Brief Description of the Drawings

Fig. 1 is a schematic diagram showing a typical shape of a cerebral aneurysm which causes subarachnoid hemorrhage.
Fig. 2 is a sectional view showing an example of an embolization device in the present invention.
Fig. 3 is a side view showing a state in which a push-out device is connected to the embolization device of the present invention.

### Reference Numerals

- 1: cerebral aneurysm
- 2: neck
- 3: parent blood vessel
- 4: embolization device
- 5: BRM coating
- 6: coil
- 7: diameter of metal wire
- 8: outer diameter of coil
- 9: connecting member
- 10: connecting means
- 11: tip
- 12: radiopaque distal section
- 13: wire section
- 14: flexible portion
- 15: proximal portion
- 16: terminal area
- 17: outer diameter of secondary coil
- 18: push-out device

### Best Mode for Carrying Out the Invention

Various embodiments of an embolization device of the present invention will be described below in detail.

Biological response modifiers (BRMs), which are proposed by the U.S. National Cancer Institute, refer to substances that modify the biological response of the host (living body) to tumors, such as cancer, or attempts to use such substances to enhance therapeutic effects. Examples of BMRs include polysaccharides and cytokines, such as interferons (IFNs), interleukins (ILs), and tumor necrosis factor (TNF). Furthermore, various clinical attempts have been made in which immunity to disease other than cancer, in particular, autoimmune disease, is enhanced by BRMs to restore homeostasis.

The present inventors have examined the wound healing response with reference to the finding that the tissue response in aneurysms treated with coil embolization follows the same pattern as that of the wound healing response. As a result, based on the fact that immunocytes, such as macrophages, play an important role in various phases in the wound healing response, an embolization device that is provided with a β(1→3) glucan as a BRM has been invented.

As described above, examples of the BRM (not according to the invention) include polysaccharides and cytokines. Since the embolization device of the present invention is a device placed in the body to embolize a vessel cavity *in vivo*, the embolization device must be sterilized when it is used. The sterilization is performed, for example, by autoclave sterilization, ethylene oxide gas sterilization, gamma-ray sterilization, or electron-beam sterilization. In consideration of the possibility that cytokines might be modified by heat or the like during sterilization, the BRM is a polysaccharide, namely a β(1→3) glucan, rather than a cytokine.

Chitin, whose chemical name is poly-N-acetyl-D-glucosamine, is a polysaccharide that constitutes the exoskeleton of crustaceans and insects, the cell membrane of fungi, etc. Chitosan is produced by deacetylation of the aminoacetyl group of chitin. It is known that if chitin or chitosan is allowed to act on damaged tissue, the production of macrophages is increased, thus increasing the number of positive cells of lysozyme, which is an important factor to promote wound healing, and the proliferation of fibroblasts is promoted, resulting in an increase in the production of collagen. Furthermore, it has been shown that chitin or chitosan improves the *in vivo* antitumor activity (NK activity and LAK activity) of lymphocytes.

According to the invention, a β(1→3) glucan is used as the polysaccharide. A polysaccharide that is a polymer containing only one type of monosaccharide is referred to as a simple polysaccharide. The β(1→3) glucan is a simple polysaccharide that is a polymer of glucose and is a polysaccharide that is contained in fruit bodies, mycelia, and cultured products of fungi. Many β(1→3) glucans have antitumor activity as BRM. It has been confirmed that some β(1→3) glucans have a branch comprising a β(1→6) glucan. Such β(1→3) glucans are also known to have antitumor activity as BRMs and are preferable as the polysaccharide. Examples of the β(1→3) glucan include curdlan and pachymaran. In view of achieving high activity as the BRM, the β(1→3) glucan is preferably curdlan. Examples of the β(1→6) glucan having the branch comprising a β(1→6) glucan include lentinan, sizofiran, sclerotan, and scleroglucan. In view of achieving high activity as the BRM, lentinan or sizofiran is preferred.

Any known method may be employed to apply the β(1→3) glucan as BRM to the embolization device. That is, the β(1→3) glucan as BRM may be applied to the embolization device by coating, adsorption, immobilization by chemical bonding, or the like. In order to maintain the activity of the β(1→3) glucan as BRM in a vessel cavity in vivo and to simplify the production process, the β(1→3) glucan as BRM is preferably applied by coating.

When the β(1→3) glucan as BRM is applied to the embolization device by coating, for example, a method in which coating is performed by spraying a solution of the β(1→3) glucan as BRM on the embolization device (spray method) or a method in which coating is performed by dipping the embolization device in a solution of the β(1→3) glucan as BRM and then withdrawing the embolization device from the solution, (dipping method) may be used. However, when a β(1→3) glucan is used as the BRM, although depending on the molecular weight of the β(1→3) glucan used, the solution of the β(1→3) glucan BRM has a relatively high viscosity, and therefore, a large scale of equipment is required in order to use the spray method. Consequently, coating by the dipping method is more preferable.

Furthermore, the embolization device may be subjected to surface treatment in order to efficiently apply the β(1→3) glucan BRM to the embolization device. The surface treatment method is not particularly limited. For example, a known method, such as coating, ultraviolet irradiation, plasma exposure, treatment with a silane coupling agent, or ion implantation may be suitably used. When any one of the surface treatment methods is performed, it is important that the β(1→3) glucan BRM be allowed to maintain its activity in a vessel cavity *in vivo.*

Such surface treatment may be performed after the β(1→3) glucan BRM is applied to the embolization device so that the embolization device is easily guided to a target vessel cavity *in vivo*. In such a case, the surface treatment method is not particularly limited. For example, a known method, such as coating, ultraviolet irradiation, plasma exposure, treatment with a silane coupling agent, or ion implantation may be suitably used. When any one of the surface treatment methods is performed, it is of course important that the BRM be allowed to maintain its activity in the vessel cavity *in vivo*.

The embolization device is intended to be used to embolize a vessel cavity *in vivo,* preferably a blood vessel, and more preferably a aneurysm formed in a blood vessel. In particular, in the case of a aneurysm, occurrence of rupture of the aneurysm during the placement of the embolization device is highly likely to lead to a very serious prognosis. Therefore, the embolization device is preferably a coil. The embolization device in the form of a coil can be flexibly deformed in the aneurysm, and the risk of rupture of the aneurysm can be significantly reduced.

The embolization device is placed percutaneously and in order to safely and rapidly embolize a vessel cavity *in vivo,* the embolization is generally performed using X-ray fluoroscopy. Consequently, the embolization device is required to be viewed by X-ray fluoroscopy. It is generally known that the visibility of a metal material in X-ray fluoroscopy improves as its density increases. Furthermore, in consideration of the workability into a coil, *in vivo* toxicity, etc., preferably, the coil comprises a metal wire composed of any one of platinum, gold, silver, and tantalum, or an alloy wire containing any one of platinum, gold, silver, and tantalum in an amount of 80% by weight or more. In the case of the alloy wire containing any one of platinum, gold, silver, and tantalum in an amount of 80% by weight or more, the type of metal to be added other than platinum, gold, silver, or tantalum is not particularly limited. By using the alloy to which a metal other than platinum, gold, silver, or tantalum is added, the physical properties of the coil can be desirably controlled. For example, by using an alloy of platinum and tungsten, it is possible to enhance the flexibility of the coil. In the platinum-tungsten alloy, the ratio of platinum is preferably 80% to 95% by weight, and more preferably 90% to 95% by weight.

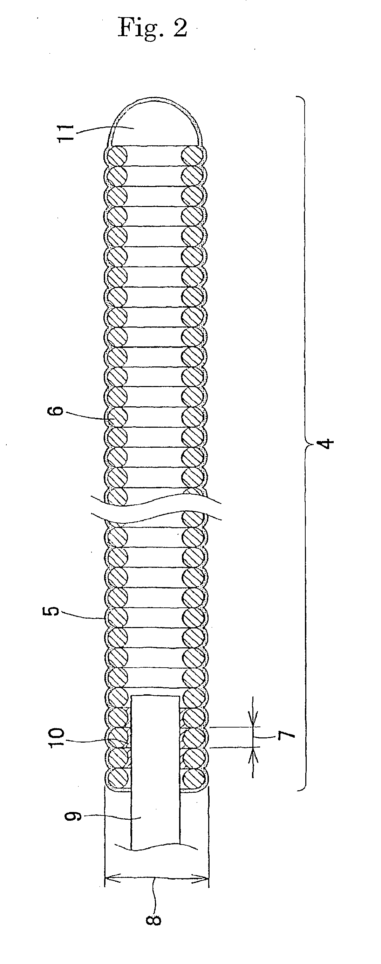

Fig. 2 is a sectional view showing an example of a structure of an embolization device 4 of the present invention. The embolization device 4 includes a coil 6, a β(1→3) glucan as BRM coating 5 provided on the coil, and a tip 11 connected to and fixed on a distal end of the coil. A connecting member 9 is fixed on a proximal end of the coil 6 by connecting means 10. The tip 11 is preferably fabricated so as to have a smooth, spherical shape from the standpoint of prevention of injury to a vessel cavity *in vivo* to be embolized.

The diameter 7 of the metal wire constituting the coil 6 is appropriately determined according to the properties of the vessel cavity *in vivo* to be embolized. Usually, the diameter 7 is preferably about 0.02 to 0.15 mm. The outer diameter 8 of the coil, which is appropriately determined for the same reason, is usually 0.1 to 1.0 mm, and preferably 0.2 to 0.6 mm.

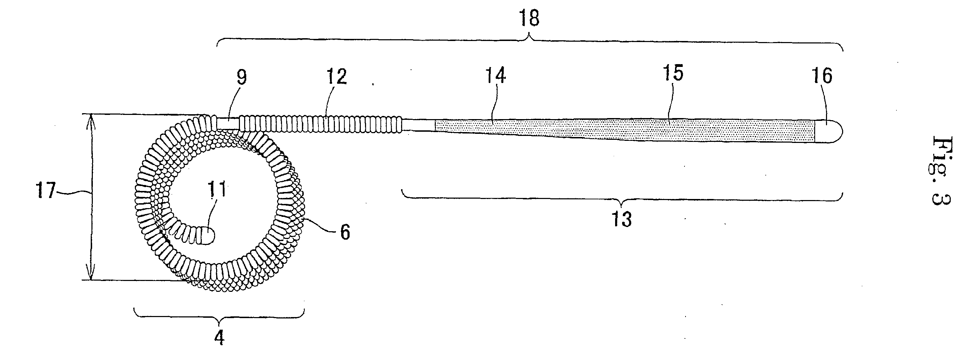

The length of the embolization device 4 is usually 1 to 1,000 mm, preferably 1 to 500 mm, and more preferably 30 to 300 mm. Fig. 2 shows the embolization device 4 that linearly stretches. The embolization device 4 has such a shape, for example, when the embolization device 4 travels through a catheter. When the embolization device 4 is not constrained by a tube wall of a catheter or the like, the embolization device 4 preferably has a secondary shape in which the coil 6 is wound as shown in Fig. 3. The secondary shape is preferably a coil shape, and the outer diameter 17 of the secondary coil shape can be appropriately selected according to the inner diameter of a vessel cavity *in vivo* to be embolized. When the vessel cavity *in vivo* to be embolized is an aneurysm, the outer diameter 17 is usually 2 to 40 mm, and preferably 2 to 20 mm. However, as the secondary shape, various shapes other than the coil shape may be selected as long as the object of the present invention is not impaired.

The properties of the coil 6 constituting the embolization device 4 do not restrict the present invention at all. Namely, a mechanism for improving the stretching strength (anti-unravel mechanism) may be provided in the coil 6. Moreover, the coil 6 is allowed to have a secondary coil shape that is suited to a vessel cavity *in vivo* to be embolized. Examples of the possible shape include a shape in which the distal portion of the secondary coil shape is curved inward and a shape in which the proximal portion of the secondary coil shape is curved inward.

Fig. 3 shows an example of a preferred assembly form in which a push-out device 18 is connected to the embolization device 4 of the present invention. The push-out device 18 shown in Fig. 3 includes a wire section 13 and a connecting member 9. The proximal portion of the rod-shaped connecting member 9 is connected to the distal end of the wire section 13, and the embolization device 4 is connected to the distal end of the connecting member 9.

In the example of the present invention shown in Fig. 3, the wire section 13 includes a proximal portion 15 covered with a coating for electrically insulating the surface thereof and a flexible portion 14 connected to the proximal portion 15, and a radiopaque distal section 12 is connected to the flexible portion. The connecting member 9 is connected to the distal end of the radiopaque distal section 12.

The outer diameter of the wire section 13 is preferably 0.1 to 2.0 mm. The length of the wire section 13 is varied according to the distance to the vessel cavity *in vivo,* and for example, is set at 0.1 to 1.8 m. The material for each of the proximal portion 15 and the flexible portion 14 is preferably a conductive metal material, such as stainless steel. For the radiopaque distal section 12, a radiopaque metal material, such as platinum, gold, silver, or tungsten, can be suitably used.

The coating provided on the proximal portion 15 can be formed using any of various known resin materials. The method for forming the coating is not particularly limited, and can be appropriately selected according to the properties of the resin material to be used. The coating is usually formed using a fluorocarbon resin material or a hydrophilic resin material. Use of a fluorocarbon resin enables a decrease in the surface friction of the proximal portion 15, which is preferable in view that the embolization device 4 can be easily guided to a target vessel cavity *in vivo.*

A terminal area 16 which is not covered with the coating and in which the metal material is exposed is formed on the proximal end of the proximal portion 15. By using any conductive member, such as a connector, a plug, or a clip, through the terminal area 16, electric power can be supplied. The length of the terminal area 16 is not particularly limited and is sufficient at about 1 to 3 cm.

The connecting member 9 may be composed of any material that does not adversely affect the living body and has characteristics in that the embolization device 4 is separated by heating. A polyvinyl alcohol-based resin which can be melt-cut by heating is suitably used for the connecting member 9. However, the material for the connecting member 9 is not limited to the polyvinyl alcohol-based resin. A material which has a property of being deformed by heating, such as a shape-memory alloy or a shape-memory resin material, can also be used. As the method for cutting off the embolization device 4 in the present invention, any of various methods can be used as long as the object of the present invention is not impaired. Examples of such methods include melt-cutting by various types of heating, melt-cutting by applying current, electrolytic cutting by applying current, and mechanical cutting (such as separation by operating a wire from outside the body or a method using a shape memory alloy).

The size of the connecting member 9 is not particularly limited, and can be appropriately set according to the sizes of the wire section 13 and the embolization device 4 to be used.

Each of the wire section 13 and the embolization device 4 is connected to and fixed on the connecting member 9. The connecting means is not particularly limited. For example, bonding using an adhesive, welding, or connection by a physical external force (swanging) may be used. In the case of bonding using an adhesive, the type of adhesive is not particularly limited, and any of various known adhesives can be used.

In one of the preferred embodiments, the assembly is guided into a vessel cavity *in vivo* through a given catheter. Specifically, a given catheter is percutaneously inserted into the living body and the distal end of the catheter is allowed to reach the vessel cavity in which the embolization device 4 is to be placed.

Subsequently, the assembly is inserted into the catheter from the embolization device 4 side. At this stage, the coil 6 constituting the embolization device 4 is moved inside the catheter with the secondary coil shape being substantially linearly stretched along the catheter. Furthermore, the embolization device 4 is allowed to protrude from the opening of the distal end of the catheter so that the connecting member 9 is positioned at the opening of the distal end of the catheter. The embolization device 4 then restores the secondary coil shape by a restoring force due to elasticity and is placed in the vessel cavity *in vivo*.

After a ground electrode is mounted on an appropriate skin surface of the living body, a high-frequency power source unit is connected to the terminal area 16, and a monopolar high-frequency current is supplied to the wire section 13. As a result, the temperature of the connecting member 9 connected to the distal end of the wire section 13 is increased by self-heating due to the high-frequency current, and the connecting member 9 is melt-cut or deformed. Consequently, the embolization device 4 is separated from the wire section 13, and thus the placement in the vessel cavity *in vivo* is completed.

For example, when a resin material comprising a polyvinyl alcohol-based copolymer is used for the connecting member 9, the embolization device 4 can be separated by supplying a high-frequency current for an extremely short period of time, e.g., within one to three seconds. The short-time separation as described above reduces the burden not only on the living body to be treated but also on the operator, which is preferable.

Examples and Comparative Example of the present invention will be described in detail below. However, it is to be understood that the present invention is not limited to the examples.

### (Example 1) - not according to the invention

A platinum-tungsten (8%) alloy wire with a wire diameter of 45 µm was wound to form a coil with an outer diameter of 300 µm and a length of 4 mm. Using dimethylacetamide (manufactured by Nacalai Tesque, Inc.) as a solvent in which 5% lithium chloride (manufactured by Nacalai Tesque, Inc.) was dissolved, a 0.5% chitin (manufactured by Wako Pure Chemical Industries, Ltd.) solution was prepared. After the coil was dipped in the 0.5% chitin solution for one minute, the coil was dipped in 2-propanol (manufactured by Nacalai Tesque, Inc.) serving as a coagulant solution for 5 minutes to coagulate the chitin solution, and thereby the surface of the coil was coated with chitin. The solvent was removed by thorough washing with distilled water, followed by drying at 60°C. An embolization device coated with chitin was thereby obtained.

### (Example 2) - not according to the invention

Using a 2% acetic acid (manufactured by Wako Pure Chemical Industries, Ltd.) aqueous solution as a solvent, a 2% Chitosan 1000 (manufactured by Wako Pure Chemical Industries, Ltd.) solution was prepared. An embolization device coated with chitosan was obtained as in Example 1 except that a 0.2 N sodium hydroxide (manufactured by Nacalai Tesque, Inc.) aqueous solution was used as a coagulant solution.

### (Example 3)

Using a 0.2 N sodium hydroxide (manufactured by Nacalai Tesque, Inc.) aqueous solution as a solvent, a 5% curdlan (manufactured by Wako Pure Chemical Industries, Ltd.) solution was prepared. An embolization device coated with curdlan was obtained as in Example 1 except that an aqueous solution containing 4% acetic acid (manufactured by Wako Pure Chemical Industries, Ltd.) and 26% sodium chloride was used as a coagulant solution.

### (Example 4)

Using a 0.5 N sodium hydroxide (manufactured by Nacalai Tesque, Inc.) aqueous solution as a solvent, 0.5% lentinan (manufactured by Yamanouchi Pharmaceutical Co., Ltd.) solution was prepared. An embolization device coated with lentinan was obtained as in Example 1 except that an aqueous solution containing 4% acetic acid (manufactured by Wako Pure Chemical Industries, Ltd.) was used as a coagulant solution.

### (Example 5)

A 1.0% sizofiran solution (Sonifilan, manufactured by Kaken Pharmaceutical Co., Ltd.) was used. An embolization device coated with sizofiran was obtained as in Example 1 except that ethanol (Nacalai Tesque, Inc.) was used as a coagulant solution and washing with distilled water was not performed.

### (Comparative Example)

A coil formed in Example 1 was used as an embolization device.

### (Evaluation of organization effect in rat simulated aneurysm)

Rats (female Wistar, 6 weeks old, 140 to 160 g) were intraperitoneally administered with, in a dose of 5 mg/rat, pentobarbital (manufactured by Dainippon Pharmaceutical Co., Ltd., Nembutal injection) to be anesthetized. In each rat, upon confirmation of deep anesthesia, the skin was incised and the left common carotid artery was exposed. The branch between the internal carotid artery and the external carotid artery was ligated, and a section 10 mm from the ligated site proximal to the heart was temporarily ligated with Schwartz clips. The blood vessel 2 mm from the peripheral ligated site proximal to the heart was incised and one of the embolization devices obtained in Examples and Comparative Example was placed in the cut. A site further proximal to the heart was ligated, and the Schwartz clips were removed. Thereby, a simulated aneurysm in which the embolization device was placed was formed. After 14 days, the rats were sacrificed and the simulated aneurysms were extirpated. After formalin fixation and paraffin embedding, circumferential cross sections were cut and hematoxylineosin (HE) staining and Elastica-van Gieson (EVG) staining were performed. The resulting sections were observed using an optical microscope, and the organization effect was evaluated.

In the HE-stained sections, in all of Examples 1 and 2 which are not according to the invention and 3 to 5, formation of large amounts of thrombi and connective tissue was observed around the placed coil and the simulated aneurysm was substantially completely embolized. In the newly formed tissue, many new blood vessels were observed and proliferation of fibroblasts was also observed. In the EVG-stained sections, production of a large amount of collagen fiber, which is believed to be derived from proliferated fibroblasts, was observed around the placed coil, and therefore, the inside of the simulated aneurysm was evaluated to be adequately organized.

In contrast, in Comparative Example, although formation of connective tissue was observed to a small extent, the inside of the simulated aneurysm was substantially completely patent. Formation of new blood vessels, proliferation of fibroblasts, and production of collagen fiber were hardly observed in the connective tissue, and therefore, the inside of the simulated aneurysm was evaluated to be insufficiently organized.

### Industrial Applicability

As described above, in accordance with the present invention, an embolization device which embolizes a vessel cavity *in vivo* and which is provided with a β(1→3) glucan as a biological response modifier (BRM) is readily provided, and it is possible to promote the organization after the embolization device is placed in the vessel cavity *in vivo*, resulting in a satisfactory embolizing effect.

## Claims

1. An embolization device for embolizing a vessel cavity *In vivo*, the embolization device comprising α β(1→3) glucan as a biological response modifier (BRM).

2. The embolization device according to claim 1 for embolizing a blood vessel.

3. The embolization device according to claim 1 for embolizing an aneurysm formed in a blood vessel.

4. The embolization device according to any one of claims 1 to 3, wherein the β(1→3) glucan as a BRM is applied by coating to a surface of the embolization device.

5. The embolization device according to any one of claims 1 to 3, wherein sold β(1→3) glucan is curdlan or pachymaran,

6. The embolization device according to any one of claims 1 to 3, wherein said β (1→3) glucan has abranch comprising a β(1→6) glucan.

7. The embolization device according to claim 6, wherein said β(1→3) glucan having a branch comprising β(1→6) glucan is selected from agroup consisting of lentinan, sizofiran, scleroten, and scleroglucan,

8. The embolization device according to claim 7, wherein said β(1→3) glucan having a branch comprising β(1→6) glucan is lentinan,

9. The embolization device according claim 7, wherein said β(1→3) glucan having a branch comprising β(1→6) glucan is sizofiran.

10. The embolization device according to any one of claims 1 to 9, wherein the embolization device is a coil,

11. The embolization device according to any one of claims 1 to 9, wherein the embolization device is a coil comprising a metal wire comprising any one of platinum, gold, silver, and tantalum, or an alloy wire containing any one of platinum, gold, silver, and tantalum in an amount of 80% by weight or more.

## Patentansprüche

1. Embolisationsvorrichtung zum Embolisieren einer Gefäßhöhle in vivo, wobei die Embolisationsvorrichtung ein β(1→3)-Glucan als biologischen Reaktionsmodifikator (BRM) umfasst.

2. Embolisationsvorrichtung nach Anspruch 1 zum Embolisieren eines Blutgefässes.

3. Embolisationsvorrichtung nach Anspruch 1 zum Embolisieren eines in einem Blutgefäß gebildeten Aneurismas.

4. Embolisationsvorrichtung nach einem der Ansprüche 1 bis 3, wobei das β(1→3)-Glucan als BRM durch Beschichtung einer Oberfläche der Embolisationsvorrichtung gewandt wird.

5. Embolisationsvorrichtung nach einem der Ansprüche 1 bis 3, wobei das β(1→3)-Glucan Curdian oder Pachymaran ist.

6. Embolisationsvorrichtung nach einem der Ansprüche 1 bis 3, wobei das β(1→3)-Glucan einen Zweig aufweist, der ein β(1→6)-Glucan umfasst.

7. Embolisationsvorrichtung nach Anspruch 6, wobei das β(1→3)-Glucan mit einem Zweig, der β(1→6)-Glucan umfasst, aus der Gruppe ausgewählt ist, bestehend aus Lentinan, Sizofiran, Sclerotan und Scleroglucan.

8. Embolisationsvorrichtung nach Anspruch 7, wobei das β(1→3)-Glucan mit einem Zweig, der β(1→6)-Glucan umfasst, Lentinan ist.

9. Embolisationsvorrichtung nach Anspruch 7, wobei das β(1→3)-Glucan mit einem Zweig, der β(1→6)-Glucan umfasst, Sizofiran ist.

10. Embolisationsvorrichtung nach einem der Ansprüche 1 bis 9, wobei die Embolisationsvorrichtung eine Spule ist.

11. Embolisationsvorrichtung nach einem der Ansprüche 1 bis 9, wobei die Embolisationsvorrichtung eine Spule ist, umfassend einen Metalldraht, umfassend eines von Platin, Gold, Silber und Tantal, oder einen Legierungsdraht, der eines von Platin, Gold, Silber und Tantal in einer Menge von 80 Gew.% oder mehr umfasst.

## Revendications

1. Dispositif d'embolisation pour l'embolisation d'une cavité vasculaire in vivo, le dispositif d'embolisation comprenant un glucane β(1→3) en tant qu'un modificateur de la réponse biologique (MRB).

2. Dispositif d'embolisation selon la revendication 1 pour l'embolisation d'un vaisseau sanguin.

3. Dispositif d'embolisation selon la revendication 1 pour l'embolisation d'un aneurisme formée dans un vaisseau sanguin.

4. Dispositif d'embolisation selon l'une des revendications 1 à 3, dans lequel le glucane ß(1→3) en tant qu'un MRB est appliqué par le revêtement d'une surface du dispositif d'embolisation.

5. Dispositif d'embolisation selon l'une des revendications 1 à 3, dans lequel ledit glucane ß(1→3) est le curdiane ou le pachymarane.

6. Dispositif d'embolisation selon l'une des revendications 1 à 3, dans lequel ledit glucane ß(1→3) a une branche comprenant un glucane ß(1→6) est le curdiane ou le pachymarane.

7. Dispositif d'embolisation selon la revendication 6, dans lequel ledit glucane ß(1→3) ayant une branche comprenant du glucane ß(1→6) est sélectionné parmi le groupe consistant en le lentinane, le sizofirane, le sclérotane et le scléroglucane.

8. Dispositif d'embolisation selon la revendication 7, dans lequel ledit glucane ß(1→3) ayant une branche comprenant du glucane ß(1→6) est le lentinane.

9. Dispositif d'embolisation selon la revendication 7, dans lequel ledit glucane ß(1→3) ayant une branche comprenant du glucane ß(1→6) est le sizofirane.

10. Dispositif d'embolisation selon l'une des revendications 1 à 9, dans lequel le dispositif d'embolisation est une bobine.

11. Dispositif d'embolisation selon l'une des revendications 1 à 9, dans lequel le dispositif d'embolisation est une bobine comprenant un fil de métal comprenant l'un quelconque de platine, d'or, d'argent, et de tantale, ou un fil d'alliage contenant l'un quelconque de platine, d'or, d'argent, et de tantale, dans une quantité de 80 % en poids ou plus.
